# EUROPEAN PATENT APPLICATION

(11) **EP 1 810 674 A1**
(43) Date of publication of application: **25.07.2007**
(21) Application number: 07380012.0
(22) Date of filing: 19.01.2007
(51) Int. Cl.: A61K 31/27, A61K 31/54, A61P 21/02, A61P 19/02, A61P 29/00, A61P 43/00

(54) **Stable pharmaceutical composition containing carisoprodol and meloxicam**

(30) Priority: 20.01.2006 MX PA06000760
(71) Applicant: Laboratorio Silanes, S.A. de C.V., Mexico, D.F. 03100 (MX)
(72) Inventor: Santiago Regalado, Antonio, 09800 Del. Iztapalapa Mexico DF (MX); Espinoza Leon, Sixto Serafin, 09800 Del. Iztapalapa Mexico DF (MX)
(74) Representative: Gil-Vega, Victor

(57) **Abstract**

The present invention provides a pharmaceutical composition or formulation in tablet form with proven stability, and its production process. This formulation combines the therapeutic action of meloxicam as an anti-inflammatory and carisoprodol as an analgesic and anti-inflammatory, which have advantageous effects when administered jointly that are not observed with the administration of the active components alone.

## Description

### Technical field of the invention:

The present invention consists of providing a pharmaceutical composition in tablet form with proven stability, and the production process thereof. These pharmaceutical compositions combine the therapeutic action of Carisoprodol and Meloxicam, a synergic effect being observed, therefore indicating a greater efficacy for pain control, as well as a reduction in some side effects.

### Background of the invention:

The present invention proposes a new stable pharmaceutical composition that is designed to jointly exploit the benefits provided by its two components, meloxicam and carisoprodol, in a combined dosage form. The joint use of the two drugs is unprecedented in the prior art, and studies carried out by our team of researchers have shown the benefits of using both components, which are more significant than when each drug is used alone. With the combination of the two drugs it is possible to achieve an unexpected effect that makes the compositions more advantageous as a medicinal product.

Specifically, the invention is based on the results achieved after carrying out phase I and II clinical trials, corresponding to the evaluation of bioavailability, safety, efficacy and potential side effects.

The closest prior art to the invention is the use of the components independently or alone, which is described below:

### Meloxicam

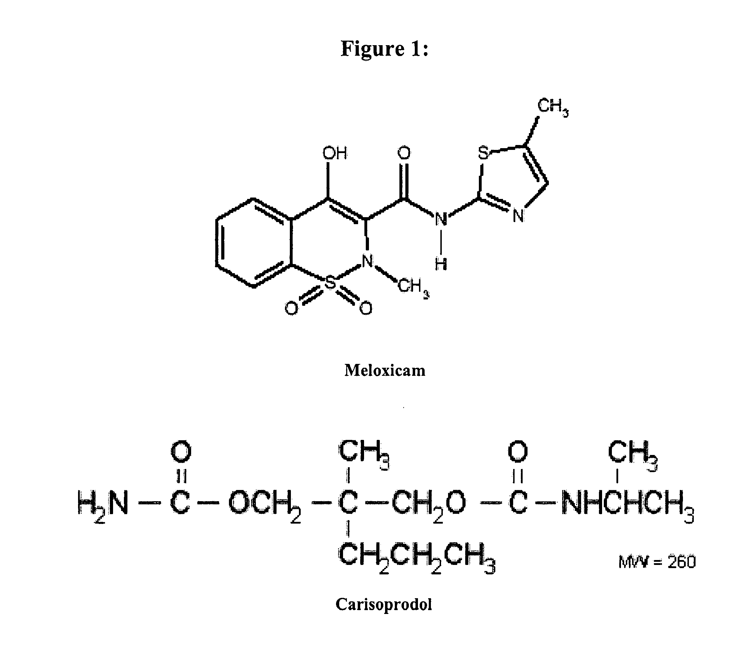

A non-steroidal anti-inflammatory drug, deriving from oxicam. It is indicated for treating the signs and symptoms of osteoarthritis and it is also used as an analgesic to relieve mild or moderate pain. Although meloxicam is often cited in the literature as a COX-2 inhibitor, it is considerably less selective for this enzyme than so-called COX-2 inhibitors. The chemical name for Meloxicam is 4-hydroxy-2-methyl-N-(5-methyl-2-thiazolyl)-2*H*-1,2-benzothiazine-3-carboxamide-1,1- dioxide. Its molecular weight is 351.4. Its empirical formula is C₁₄H₁₃N₃O₄S₂ and it has the structural formula shown in figure 1. It is a solid that is practically insoluble in water, with a high degree of solubility observed in strong bases and acids. It is very slightly soluble in methanol. It has an apparent partition coefficient of (log P)ₐₚₚ= 0.1 in *n*-octanol/buffer pH 7.4. Meloxicam has pKa values of 1.1 and 4.2.
Mechanism of Action: Like other non-steroidal anti-inflammatory drugs, meloxicam inhibits COX-1 and COX-2 enzymes (cyclooxygenases). These enzymes catalyse the conversion of arachidonic acid to prostaglandin G2, which in turn is a precursor of other prostaglandins and thromboxane A2, whilst COX-2 favours the synthesis of prostaglandins that have a physiological function on the gastric mucosa and renal activity.

Meloxicam is more selective towards COX-2 than indometacin, but less than selective inhibitors of this enzyme such as celecoxib and rofecoxib. It does not inhibit platelet aggregation induced by collagen or arachidonic acid but it does significantly reduce the production of thromboxane in the platelets.
Pharmacokinetics: It is rapidly and almost completely absorbed after (oral) administration, up to 89%, without food interfering in said process. It reaches its maximum concentration between 4 and 11 hours after administration. Its half-life is between 15 and 20 hours, undergoing enterohepatic circulation, as a second peak is observed with maximum concentrations 12-14 hours after administration.

99.5% binds to plasma proteins, with a volume of distribution of 13 to 16 litres. It undergoes hepatic metabolism by oxidation, using CYP3A4, which gives rise to four inactive metabolites. Its main metabolite is formed by the action of cytochrome P450, in particular the isoenzyme CYP3A4.

43% of it is eliminated through the urine in the form of metabolites, while the rest is excreted through faeces, with a total clearance of 0.4 to 0.5 1/h. Meloxicam therefore shows a linear pharmacokinetic profile, reaching a steady state after five days of doses. Women present lower plasma concentrations than men of the same age.

### Carisoprodol

It is a skeletal muscle relaxant drug that is chemically related to meprobamate. This medicine has proved to be effective in treating secondary skeletal muscle spasms.

Carisoprodol was evaluated in 1980 (Elenbass, J.K. Centrally acting oral skeletal muscle relaxants. Am J. Hosp. Pharm., 1980 37:1313-1323) with subjective measurements and a suitable design, which did not enable the authors to establish the superiority of any particular muscle relaxant. However, what did become clear was its lower degree of efficacy in chronic problems compared to acute cases. It has the formula shown in figure 1.
**Mechanism of action:** It is not known exactly how it works. However, this drug preferably depresses polysynaptic reflexes and at higher doses monosynaptic reflexes (Elenbass, 1980), although sedation is important, particularly in high doses.
**Pharmacokinetics:** It is adequately absorbed in the gastrointestinal tract, reaching its maximum concentration within 2 to 4 hours, although the initial muscle skeletal relaxant effect is observed after 30 to 45 minutes.

It is distributed extensively and is metabolised hepatically to meprobamate and hydroxymeprobamate by the enzyme CYP 2C19. It is mainly eliminated through the urine in the form of meprobamate. It has an elimination half-life of 8 hours.

Its use is not recommended for pregnant patients or while breastfeeding. Likewise, it is recommended that this product should not be used in patients who handle machinery.

### Objects of the invention:

A general object of the present invention is to provide a new composition for oral administration containing a COX II inhibitor drug and muscle relaxant, which, according to the composition of the pharmaceutical form, is capable of releasing the COX II inhibitor at approximately the same rate as the relaxant, both in less than an hour.

More particularly, the present invention relates to a new use given to a COX II inhibitor such as meloxicam and a muscle relaxant such as carisoprodol, which, when they act jointly, present a synergic or enhanced effect that is neither obvious nor derivable from the prior art, and this effect promotes more effective pain control than when these components are used along in patients who require it.

The present invention also relates to a new use given to a COX II inhibitor such as meloxicam and carisoprodol, which, when they act jointly, present a reduction in the known side effects that the components present in patients when administered and acting alone.

A further object of the present invention is to provide a pharmaceutical composition wherein, after oral administration, both drugs, meloxicam and carisoprodol, are released almost immediately in aqueous media, more particularly in less than 1 hour.

More particularly, the new pharmaceutical composition is in tablet form, with proven stability, in a unitary dosage containing two drugs, with carisoprodol in a quantity of 200 mg to 600 mg and meloxicam in a quantity of 7.5 mg to 15 mg, both to be released simultaneously and immediately.

Another object of the present invention is to provide a pharmaceutical composition in tablet form that can be administered orally, preferably in one or more doses per day depending on the patient's requirements.

Yet another of the objects of the present invention is to provide a pharmaceutical composition that combines two active substances, wherein it also contains at least one of the following excipients: povidone K 30, which acts in the composition as both a binder and solubilising agent; Crospovidone, which acts as a disintegrant; Sodium citrate, as a pH-regulating agent, colloidal silicon dioxide, as a glidant; sodium lauryl sulphate, which acts as a solubilising agent; microcrystalline cellulose PH 101, as a filler together with lactose monohydrate; magnesium stearate, as an antiadherent; and the product can be prepared with or without purified water.

Another of the objects of the present invention is to provide a pharmaceutical composition that includes one or more of the following active drugs: a) a COX-II inhibitor, where the COX-II inhibitor was selected from a group consisting of rofecoxib, celecoxib, flosulide, meloxicam, nabumetone, etodolac, nimesulide, b) a muscle relaxant selected from the group consisting of alcuronium, alosetron, aminophyline, baclofen, carisoprodol, methocarbamol.

### Detailed description of the invention:

The present invention provides a pharmaceutical composition that can be used in the treatment of muscular or skeletal problems, such as neck pain, twists, sprains, dislocations, sport-related and accidental injuries, muscle, tendon and ligament strain, spinal pain syndromes (lower back pain, sciatica, etc), posttraumatic and postoperative pain, fractures and other processes accompanied by inflammation and muscle contraction. It can also be used to relieve acute episodes of certain types of rheumatic pain (osteoarthritis, arthrosis, extraarticular rheumatism, ankylosing spondylitis, signs and symptoms of rheumatoid arthritis).

As part of the overall therapy, it might be advisable to recommend an alternative treatment such as hydrotherapy, physiotherapy, etc., depending on each case.

The present description relates to a new invention that consists of pharmaceutical forms of proven stability in a unitary dosage in tablet form containing two drugs for immediate release, the concentration of which may vary according to the dose, although in trials the tablets preferably contain 200 mg of carisoprodol, which acts as the muscle relaxant, and either 7.5 mg or 15 mg of meloxicam, which is a type of COX II inhibitor.

The present invention has full technical support given the results observed in phase II clinical trials relating to the efficacy of the pharmaceutical formulations designed. In the proof of concept study, the efficacy of administering a single dose of meloxicam and carisoprodol simultaneously in the same formulation was compared to a placebo and meloxicam alone, as is described in the examples section.

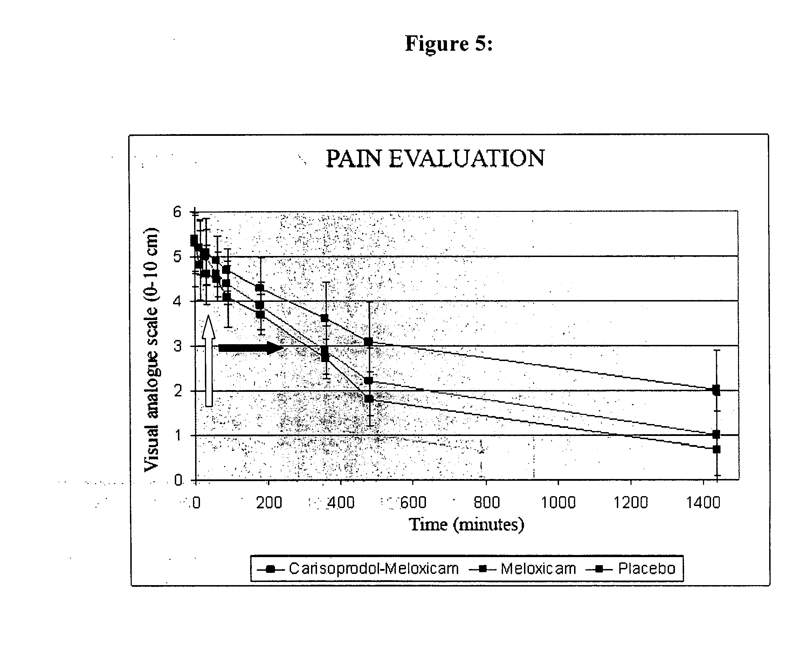

Said phase II clinical trials, (see examples below) gave the results presented below in patients with grade I and II sprains, which, although they recover on their own by applying the general measures that are recommended, such as applying an ice pack, rest, elevation of the injured limb and bandaging, show that administration of drugs increases the effect of these physical measures. Another discovery of these trials was that the administration of carisoprodol-meloxicam at doses of 200 and 15 mg respectively, as is shown by the results of example 5 of the present description (see figure 5), brings about a significant analgesic effect 90 minutes after its administration (white arrow in figure 5), and meloxicam alone takes longer in bringing about an analgesic effect, up to 360 minutes after its administration (dark arrow in figure 5). It has also been found that the carisoprodol-meloxicam combination helps functional recovery, i.e. the ability to put weight on the injured limb, 30 minutes after its administration. This contrasts with the group that only received meloxicam, as the analgesic effect and the effect of functional recovery are observed later in the latter group. It has been noted that administration of carisoprodol alone can produce analgesia in patients with lower back pain and also in patients with dental pain.

From the phase 1 clinical trials carried out, it was also found that, on comparing the carisoprodol-meloxicam formulation with meloxicam as a monodrug, a statistically significant reduction was observed in the erythrocyte levels in faeces, haematocrit and haemoglobin values, which might be a sign that it causes a lower degree of intestinal bleeding.

Furthermore, from the point of view of symptoms, the patients who received the combination reported adverse effects mainly corresponding to carisoprodol, although at the tested dose of carisoprodol (200 mg) no changes were noted in the psychological tests, which suggests that this dose is safe and does not alter the reaction time or cognition of the subject, enabling the patient to continue with normal daily activities, unlike the effects reported for higher doses of carisoprodol. As regards the reduction in arterial pressure observed with the single dose of carisoprodol and with the carisoprodol-meloxicam combination, it could be said that this might reflect the anxiolytic action of carisoprodol. Moreover, although this effect was present in the volunteers who received the drug for 15 days, it is no different from the other treatments.

It was concluded that, in general terms, the combination of carisoprodol-meloxicam in the same formulation is safe for administration in short courses (15 days), as it does not cause intestinal damage and does not cause a reduction in the response time or in factors such as attention and concentration, as is the case with other formulations on the market.

In general, the characteristics of the drugs are very different, which causes problems during the manufacturing process to produce a product with a uniform drug content.

The present invention proposes a new stable pharmaceutical composition that is designed to contain combinations of two active substances in ranges that make it possible to control muscular or skeletal problems with one or two oral administrations per day. The pharmaceutical composition contains the two biologically active agents in a single phase and in a homogeneous composition, presenting substantial differences from one another in terms of their inherent physicochemical properties, as well as being required in proportions that represent a significant disproportion with regard to concentration, weight and volume.

The new pharmaceutical composition contains Meloxicam as its active substance, a benzothiazine carboxamide derivative that in turn is derived from enolic acid, and it is combined with a second active substance, carisoprodol, which is a skeletal muscle relaxant that is chemically related to meprobamate. This drug has proved to be effective in treating secondary discomfort resulting from skeletal muscle contraction, and as an adjuvant to rest in physiotherapy.

As is described in detail, despite the differences in terms of proportion and physicochemical properties, it is a composition with a pharmaceutically suitable stability, as there is no possibility of the two drugs becoming degraded during storage and no problems of incompatibility that delay the release of each of drug were observed during their release, in accordance with the desired parameters, even when different concentrations were used (7.5 to 15 mg of Meloxicam and 200 to 600 mg of Carisoprodol). The pharmaceutical composition of the invention presents a rapid dissolution of both drugs, which are released in less than one hour.

In dissolution tests carried out on the product, the releases observed were similar to those found in commercial products with only one of the drugs, i.e. in tablets containing only meloxicam and in tablets containing only carisoprodol. For both drugs the releases were about 95%, respectively (see table 2). It can be deduced from this data that, thanks to its composition, the tablet of the present invention enables the immediate release of both drugs without either of them competing for greater dissolution.

The production process of said composition is also described as part of the invention, which, from the technical point of view, has a very acceptable degree of laboriousness or difficulty in industrial-scale manufacturing, resulting in advantages in terms of production costs.

### Production process of the granulate containing carisoprodol:

The manufacturing process of the granulate with the components of the formula for preparing the granules containing carisoprodol consisted of screening Carisoprodol, Povidone K 30, 50% of Crospovidone, 50% of Microcrystalline cellulose PH 101, Lactose monohydrate, Sodium lauryl sulphate and 75% of Colloidal silicon dioxide, through 15 to 60 mesh.

The screened material from the previous step is mixed in a Collette Ultima type granulator for 5 to 20 minutes using the main mixer at a speed of 50 to 150 rpm and the high-shear mixer at a speed of 300 to 600 rpm. The powdered mixture is wetted with Purified water, at the following settings: 5 minutes using the main mixer at a speed of 70 to 120 rpm and the high-shear mixer at a speed of 300 to 700 rpm. The granulation liquid is added at an average rate of 7 to 15 ml/sec. The heat balance system of the granulation vessel keeps the vessel and the lid at 30°C. The powder is granulated at the following settings: 5 to 10 minutes using the main mixer at a speed of 120 rpm and the high-shear mixer at a speed of 900 rpm. The heat balance system of the granulation vessel keeps the vessel and the lid at 30°C.

The resulting granulate is dried in gradual stages with the following settings: 5 to 10 minutes at a speed of 20 to 65 rpm in the main mixer and at a speed of 900 rpm in the high-shear mixer. The vacuum system maintains a value of less than 80 mbar inside the granulation vessel and the heat balance system keeps the lid at 60°C. The aforementioned settings are maintained for 10 minutes and then the microwave system is switched on and run at 50 % of its maximum power (0.5 KW). After 20 minutes the equipment's settings are changed, the microwave being set to 75 % of its maximum power (0.75 KW) and the movement system allowing the granulation vessel to swing to an angle of 45° at its highest point.

After the previous step, the equipment's settings are changed for 25 minutes to 100% of the microwave's power, whilst all the other parameters remain the same. The process continues until the humidity of the granulate lies within a range of 2.0-3.0 per cent.

### Production process of the tablets.

The following ingredients are separately placed in a Gallay Systems type laboratory mixer: Meloxicam, 25% of Colloidal silicon dioxide, 50% of Crospovidone, and 50% of Microcrystalline cellulose PH 101, and they are mixed for 5 minutes at 12 rpm. The mixture resulting from the previous step is screened through 2A062R-calibre mesh using Quadro Comil type milling equipment at an impeller speed of 1200 rpm.

The screened granulate resulting from the previous step is then placed in the Gallay type mixer together with the mixture containing the carisoprodol granulate and they are mixed for no less than 25 minutes at 12 rpm.

The micronised Magnesium stearate is separately screened through 2A062R-calibre mesh, using the Quadro Comil type mill at an impeller speed of 1200 rpm. The screened powder from the previous step is added and they are mixed for 5 minutes at 12 rpm.

The powdered mixture is pressed to specifications using 17.0 x 7.2 mm punches in a GEA Courtoy Modul type tablet press.

The tablets can be packaged in different primary packaging, blister packs (PVC, PVDC, PET) and bottles (HDPE, Glass, PET), which are subjected to stability studies under accelerated conditions following the international guidelines established in the USP (United States Pharmacopoeia 28- National Formulary 23. Rockville, MD: U.S. Pharmacopoeial Convention 2005) and the ICH (International Conference of Harmonisation, Guideline for Industry, Stability Testing of New Drug Substances and Products. ICH-QIA September 1994).

In the invention disclosed herein, it is considered that all optional modifications that a person skilled in the art may choose or carry out lie within the scope of the present invention.

### Description of the figures

**Figure 1:** Structural formula of meloxicam and carisoprodol.

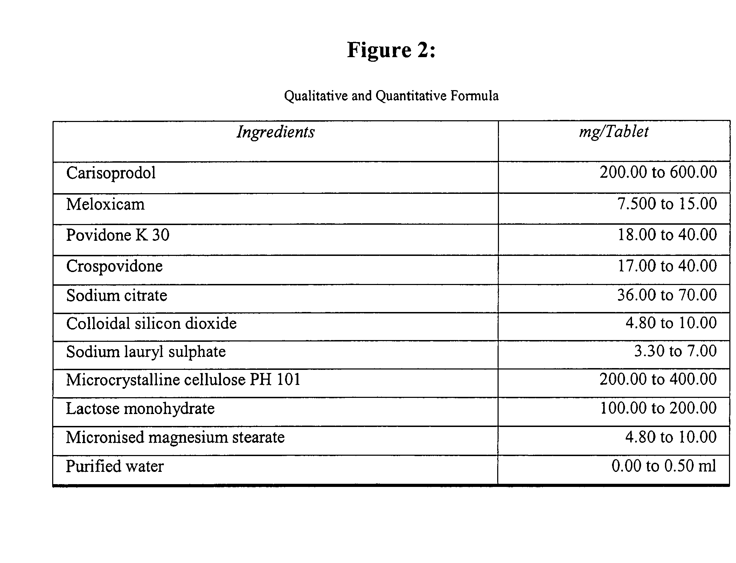

**Figure 2:** Qualitative and quantitative formulation of the composition of the invention.

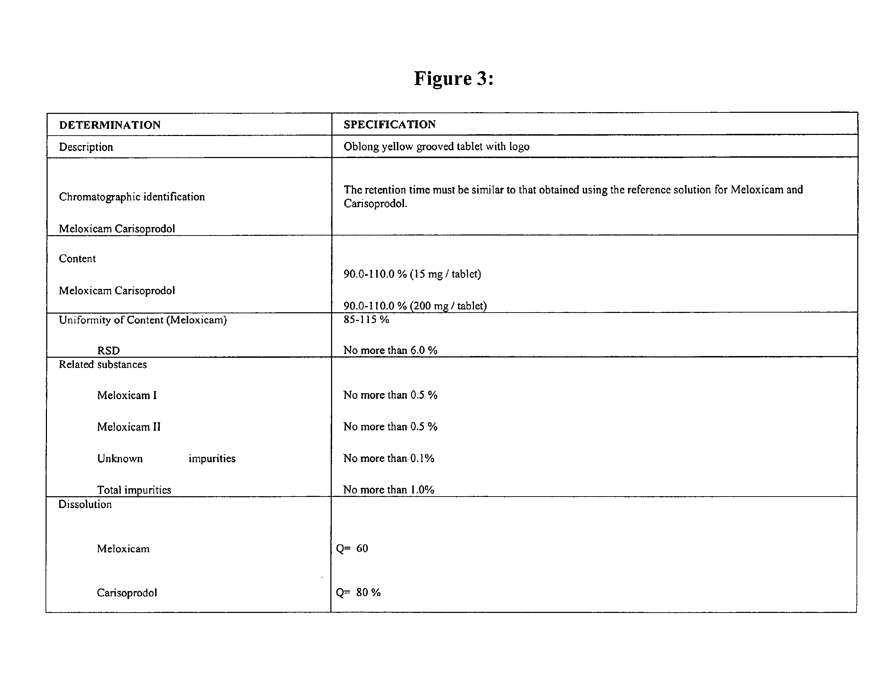

**Figure 3:** Description of the finished product once the steps mentioned in example 1 of the present description have been carried out.
**Figure 4:** Monitoring of Stability Studies for the product Dorsal 15/200 mg Tablets.
**Figure 5:** Analgesic efficacy of the treatments. The graph shows the effect-time relation for three different treatments. Each point represents the response of 10 patients with pain caused by a sprained ankle. As can be seen, for the case of the carisoprodol-meloxicam combination the effect is statistically significant compared to the placebo (p=0.022) after 90 minutes (white arrow) and the significance increases until 1440 minutes (p=0.0020), whilst with meloxicam the reduction in VAS is statistically significant from minute 360 (p=0.021) (dark arrow) and it is maintained until minute 1440.

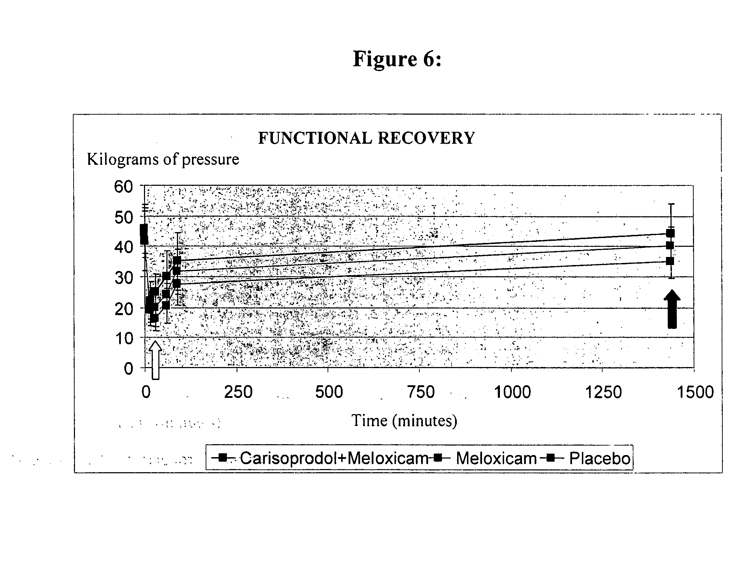

**Figure 6:** Analgesic efficacy of the different treatments. The graph shows the effect-time relation for three different treatments. Each point represents the response of 10 patients in the functional recovery of patients with pain caused by a sprained ankle. As can be seen, for the case of the carisoprodol-meloxicam combination the effect is statistically significant compared to the placebo (p=0.0004) after 30 minutes (white arrow) and the significance increases until 1440 minutes (p=0.008), whilst with meloxicam the recovery is statistically significant until minute 1440 (p=0.037) (dark arrow).

### Examples

The following examples are not intended to limit the scope of the invention, being merely illustrative and showing the full experimental support that is appropriate information in the technical field of the invention. The examples establish an embodiment of the pharmaceutical composition and an embodiment of the method for producing said tablets, as well as providing an example of the positive results of the stability studies and phase I and II clinical trials.

### EXAMPLE 1:

The production process of the pharmaceutical composition of the invention, in tablet form, which essentially consists of the following steps:
a) mixing Carisoprodol, Povidone K 30, 50% of Crospovidone, 50% of Microcrystalline cellulose PH 101, Lactose monohydrate, Sodium lauryl sulphate and 75% of Colloidal silicon dioxide, and screening them through 15 to 60 mesh.
b) mixing the screened ingredients from the previous step in a Collette Ultima type granulator for 5 to 20 minutes using a high-shear mixer at a speed of 300 to 600 rpm.
c) wetting the powdered mixture with purified water, in single-step granulation equipment (Ultima type) at a rate of 7 to 15 ml/sec.
d) drying the resulting granulate in gradual stages in the same processing equipment for approximately 60 minutes, the lid having a heat balance between 50° and 70° C and a pressure of less than 80 mbar, with nitrogen gas injected into the system and application of microwaves, according to the instructions provided with the processing equipment that is used; until its humidity lies within a range of 2.0-3.0 per cent.
e) mixing Meloxicam, 25% of Colloidal silicon dioxide, 50% of Crospovidone, 50% of Microcrystalline cellulose PH 101 for 5 minutes at 12 rpm, type of equipment: Gallay Systems.
f) screening the mixture through 2A062R-calibre mesh using Quadro Comil type screening equipment at an impeller speed of 1200 rpm.
g) mixing the mixture from the previous step and the mixture containing the carisoprodol granulate in Gallay Systems Laboratory equipment for no less than 25 minutes to 12 rpm.
h) separately screening micronised Magnesium stearate through 2A062R-calibre mesh using a Quadro Comil type mill at an impeller speed of 1200 rpm and adding the screened powder resulting from the previous step and mixing for 5 minutes at 12 rpm.
i) pressing the resulting powder mixture according to the established specifications using 17.0 x 7.2 mm punches in a GEA Courtoy Modul type tablet press.

The tablets can be packaged in different primary packaging, blister packs (PVC, PVDC, PET) and bottles (HDPE, Glass, PET), which are subjected to stability studies under accelerated conditions following the international guidelines established in the USP (United States Pharmacopoeia 28- National Formulary 23. Rockville, MD: U.S. Pharmacopoeial Convention 2005) and the ICH (International Conference of Harmonisation, Guideline for Industry, Stability Testing of New Drug Substances and Products. ICH-Q1A September 1994).

### EXAMPLE 2:

Presentations formulated for the finished product

| Proportion of Meloxicam | Carisoprodol |
|---|---|
| 7.5 mg | 200 mg |
| 15 mg | 200 mg |

### EXAMPLE 3:

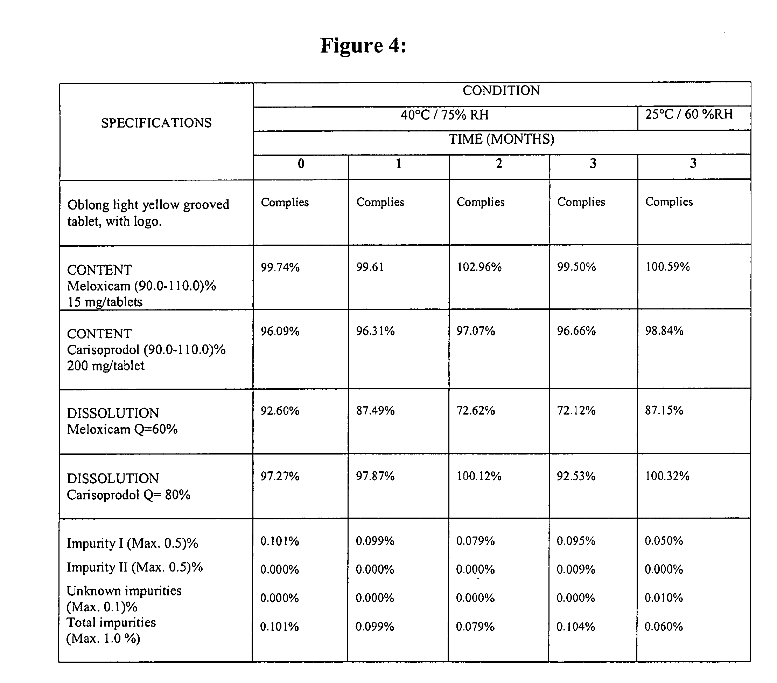

The stability studies carried out on the pharmaceutical compositions manufactured as described in the present invention were performed under selected temperature and humidity conditions, which were 40 °C and 75 % relative humidity and 25°C and 60% relative humidity. The study was carried out over 3 months, proving that the pharmaceutical composition is stable, as can be seen from the results shown in figure 4.

### EXAMPLE 4:

A Phase I trial was carried out on the bioavailability, safety and tolerability of meloxicam and carisoprodol administered in the same formulation and separately. 60 healthy male volunteers took part in the trial, and they were divided into four treatment groups. The following compounds were administered: Treatment A (Tablets containing a combination of 200 mg of carisoprodol and 15 mg of meloxicam), treatment B (Tablets containing 15 mg of meloxicam), treatment C (Tablets containing 200 mg of carisoprodol), and treatment D (placebo). It was a prospective, single-blind, parallel, longitudinal, comparative, placebo-controlled trial with the components of the combination also administered separately and with the subjects randomly distributed into 4 groups. The bioavailability was studied after administration of a single dose of the medicines under investigation and also during the steady state (after 15 days of administration of the medicines). In the case of meloxicam and carisoprodol, by determining the pharmacokinetic parameters of Cmax and area under the plasma concentration curve in relation to time (ABCo-t and ABCo-inf), it is possible to characterise the bioavailability of the drug from its pharmaceutical form, in terms of the rate and degree of absorption respectively. The application of statistical tests on data transformed to natural logarithm established that there are no statistically significant differences between treatments when evaluating the pharmacokinetics of the initial dose; whilst the multiple-dose scheme at steady state showed an apparent difference in most of the parameters that were evaluated, where the plasma levels reached by treatment A (meloxicam combined with carisoprodol) were generally lower than those shown for treatment B (meloxicam alone). In the case of single-dose meloxicam, either combined with carisoprodol or individually, the probability quotients that the values would be below or above the permitted limits were always lower than 5% (p < 0.05). However, in the case of repeated administrations of meloxicam the values were above 5%. Therefore, it can be concluded that in acute administration meloxicam is bioequivalent, whether combined or alone. In the case of carisoprodol, there is no bioequivalence in any case. This indicates that, in terms of bioavailability, the combination of meloxicam 15 mg plus carisoprodol 200 mg in a formulation is different to the individual components, which also suggests that the combination modifies the bioavailability of the components or that pharmaceutical aspects influence the quantity of drug that is absorbed, especially with repeated administration.

Moreover, with regard to the tolerability and safety studies, the present clinical trial indicates that the combination of meloxicam-Carisoprodol 15/200 mg is at least as tolerable as meloxicam alone and carisoprodol alone.

### EXAMPLE 5:

### Single-dose pharmacokinetics study

The volunteers were administered the randomly assigned drug and blood samples were taken at the following times: 0, 15, 30, 60 and 90 min, and 2, 3, 4, 4.5, 5, 5.5, 6, 8, 12 and 24 hours after administration of the drug.

### Stable-state pharmacokinetics study

Type of study: three-arm, parallel, randomised, prospective, longitudinal, single-blind, balanced.
Group A: received the combination of 200 mg of carosiprodol and 15 mg of meloxicam
Group B: Received 15 mg of meloxicam
Group C: Received 200 mg of carisoprodol

They continued for 15 days with the administration of a daily dose of medicine. A pharmacokinetics study was carried out on day 1 and day 14 in order to clearly identify the bioavailability in a single dose and a multiple dose. The bioequivalence of the combined product was also compared with that of the individual products.

It was found that the maximum concentration (Cmax) is similar in the meloxicam-carisoprodol combination and in the administration of meloxicam alone at a dose of 15 mg, both in a single dose and at a maintenance dose (bioequivalence). It was found that there was gastrointestinal tolerability, as measured by endoscopy, and no significant differences were observed in the gastric mucosa by comparing the meloxicam-carisoprodol combination with a placebo. As regards the sedative effect of Carisoprodol, no significant differences were observed between the MINIMENTAL results prior to administration of the medicine and the MINIMENTAL results subsequent to administration. This was the case with both the administration of carisoprodol alone at a dose of 200 mg and the meloxicam-carisoprodol combination. As regards gastrointestinal bleeding, a statistically significant reduction in haematocrit and haemoglobin was found with the administration of meloxicam alone at a dose of 15 mg and these changes were not seen when the combination meloxicam-carisoprodol was administered.

### EXAMPLE 6:

**Pain evaluation by V.A.S.** It was a prospective, single-blind, parallel, longitudinal trial with 30 male patients who had given informed consent and who had suffered a grade I or grade II ankle sprain type injury whilst playing sport. They were randomly divided into three treatment groups: treatment A: tablets containing 200 mg of Carisoprodol and 15 mg of meloxicam; treatment B: tablets containing 15 mg of meloxicam; treatment C: placebo. The treatment with physical measures significantly reduced the pain classification on the VAS scale in patients who were given the placebo, although this effect was no complete. However, the administration of meloxicam-carisoprodol or meloxicam brought about a statistically significant reduction in the classification on the VAS scale, leading to scores of less than one (see the graph in figure 5).

### EXAMPLE 7:

**Functional recovery.** Functional recovery was achieved in all the cases, whether the patient was treated with meloxicam-carisoprodol or meloxicam or the placebo. This effect was fastest in the group that was administered meloxicam-carisoprodol. In fact, if this group is statistically compared with the placebo group, it can be seen that it is different from minute 30, whilst if the group that was administered meloxicam is compared with the placebo group, it is statistically different up to 24 hours, (see the graph in figure 6).

For a person skilled in the art of the invention it will be possible to use the examples described above, substituting the components that make up the excipient in formulations, including suitable excipients to formulate oral suspensions, which can include e.g. colloidal silicon dioxide, hydroxymethylcellulose, sorbitol, glycerol, xylotol, monobasic sodium phosphate, sodium saccharine, sodium benzoate, citric acid, artificial flavourings and purified water. Therefore, the additives, excipients, proportions of the components, formulation methods and other parameters described herein can be modified or substituted for a variety of options, always bearing in mind the description.

## Claims

1. A stable pharmaceutical composition, **characterised in that** it contains a combination of a COX II inhibitor and a muscle relaxant, both for immediate release.

2. The pharmaceutical composition of claim 1, **characterised in that** it is in tablet form.

3. The pharmaceutical composition of either of claims 1 or 2, **characterised in that** the COX II inhibitor is Meloxicam and the muscle relaxant is Carisoprodol.

4. The pharmaceutical composition of any of claims 1, 2 or 3, **characterised in that** it contains an equivalent quantity of 7.5 to 15 mg of Meloxicam and it contains an equivalent quantity of 200 to 600 mg of Carisoprodol.

5. The use of meloxicam and carisoprodol to prepare a medicine for treating muscular or skeletal problems, such as neck pain, twists, sprains, dislocations, sport-related and accidental injuries, muscle, tendon and ligament strain, spinal pain syndromes (lower back pain, sciatica, etc), posttraumatic and postoperative pain, fractures and other processes accompanied by inflammation and muscle contraction, and in acute episodes of certain types of rheumatic pain selected from: osteoarthritis, arthrosis, extra-articular rheumatism, ankylosing spondylitis, signs and symptoms of rheumatoid arthritis.

6. The use according to claim 5, **characterised in that** said medicine is a tablet containing 200 mg of carisoprodol and 15 mg of meloxicam, which can be administered in one or two doses per day.

7. The use according to claims 5 or 6, wherein said medicine brings about a reduction in pain and functional recovery from minute 90 after administration.

8. The use according to any of claims 5, 6 or 7, wherein said medicine brings about a greater reduction in gastrointestinal bleeding than that observed when meloxicam is administered separately.
